Europäisches Patentamt

European Patent Office

Office européen des brevets

(11) Publication number: **0 532 706 B1**

# EUROPEAN PATENT SPECIFICATION

(45) Date of publication of patent specification: **10.05.95**  (51) Int. Cl.6: **A61K 31/34**, A61K 31/195, A61K 31/375

(21) Application number: **91915232.2**

(22) Date of filing: **03.06.91**

(86) International application number:
**PCT/US91/03876**

(87) International publication number:
**WO 91/19488 (26.12.91 91/29)**

(54) **REDUCTION OF CARDIOVASCULAR VESSEL OCCLUSIONS WITH ASCORBATE AND LIPOPROTEIN (a) BINDING INHIBITORS.**

(30) Priority: **04.06.90 US 533129**
**24.07.90 US 556968**
**24.07.90 US 557516**

(43) Date of publication of application:
**24.03.93 Bulletin 93/12**

(45) Publication of the grant of the patent:
**10.05.95 Bulletin 95/19**

(84) Designated Contracting States:
**CH DE FR GB IT LI**

(56) References cited:
**JP-A-60 078 560**
**US-A- 4 954 521**

**Week 8921, Derwent Publications Ltd., London, GB; AN 89-153910**

**NUTRITION REVIEWS vol. 25, no. 6, 1967, pages 183 - 185 'Ascorbic acid effecton blood lipids and lipoprotein lipase'**

(73) Proprietor: **THERAPY 2000**
**880 Bear Gulch Road**
**Woodside, CA 94062 (US)**

(72) Inventor: **Rath, Matthias**
**Eberhardstrass 12**
**W-7141 Kirchberg/Murr (DE)**
Inventor: **Pauling, Linus C.**
**15 Salmon Creek**
**Big Sur, CA 93920 (US)**

(74) Representative: **Schmitz, Jean-Marie et al**
**OFFICE DENNEMEYER S.à.r.l.**
**55, rue des Bruyères**
**L-1274 Howald (LU)**

W.FORTH ET AL. 'Allgemeine und spezielle Pharmakologie und Toxikologie' 1980 ,B.I.WISSENSCHAFTSVERLAG , MANNHEIM, GERMANY

Martindale, The Extra Pharmacopoeia, 28th edition, (1982), pg. 56, column 2.

The Nutrition Desk Reference, GARRISON et al., Keats Publishing, Inc., (1985),pp 172-77, note page 173-175, column 1.

Vitamin C In Health And Disease, BASU et al., AVI Publishing Co., Inc., (1982),pp 95-101, note pg. 98, column 1.

CHEMICAL ABSTRACTS, 77(13): 86318, (1972), note entire Abstract.

**Description**

The present invention relates to the use of a composition comprising ascorbate and tranexamic acid for the manufacture of a medicament for therapeutic application.

The present invention relates generally to the prevention and treatment of cardiovascular and related diseases and more particularly to compounds that inhibit the binding of lipoprotein (a) to components of the arterial wall.

Lipoprotein (a) ("Lp(a)") was first identified by Blumberg, B.S., et al. (1962) *J. Clin. Invest.* 41:1936-1944 and Berg, K. (1963) *Acta Pathol.* 59:369-382. The structure of Lp(a) resembles that of low-density lipoprotein ("LDL") in that both share a lipid/apoprotein composition, mainly apolipoprotein B-100 ("apo B"), the ligand by which LDL binds to the LDL receptors present on the interior surfaces of arterial walls.

The unique feature of Lp(a) is an additional glycoprotein, designated apoprotein (a) ("apo(a)"), which is linked to apo B by disulfide groups. The cDNA sequence of apo(a) shows a striking homology to plasminogen, with multiple repeats of kringle 4, one kringle 5, and a protease domain. The isoforms of apo-(a) vary in the range of 300 to 800 kDa and differ mainly in their genetically determined number of kringle 4 structures (McLean, J.W., et al. (1987) *Nature* 300:132-137). Apo(a) has no plasmin-like protease activity (Eaton, D.L., et al., (1987) *Proc. Natl. Acad. Sci. USA* 84:3224-3228). Serine protease activity, however, has been demonstrated (Salonen, E., et al. (1989) *EMBO J.* 8:4035-4040).

Like plasminogen, Lp(a) has been shown to bind lysine-sepharose, immobilized fibrin and fibrinogen, and the plasminogen receptor on endothelial cells (Harpel, P.C., et al. (1989) *Proc. Natl. Acad. Sci. USA* 86:3847-3851; Gonzalez-Gronow, M., et al. (1989) *Biochemistry* 28:2374-2377; Miles, L. et al. (1989) *Nature* 339:301-302; Hajjar, K.A., et al. (1989) *Nature* 339:303-305). Furthermore, Lp(a) has been demonstrated to bind to other components of the arterial wall like fibronectin and glycosaminoglycanes. The precise nature of these bindings, however, is poorly understood.

Essentially all human blood contains Lp(a). There can, however, be a thousand-fold range in its plasma concentration between individuals. High levels of Lp(a) are associated with a high incidence of cardiovascular disease (Armstrong, V.W., et al. (1986) *Atherosclerosis* 62:249-257; Dahlem, G., et al. (1986) *Circulation* 74:758-765; Miles, L.A., et al. (1989) *Nature* 339:301-302; Zenker, G., et al. (1986) *Stroke* 17:942-945). The term cardiovascular disease will be used hereafter as including all pathological states leading to a narrowing and/or occlusion of blood vessels throughout the body, but particularly atherosclerosis, thrombosis and other related pathological states, especially as occurs in the arteries of the heart muscle and the brain.

It has also been suggested that Lp(a), the concentration of which increases markedly in the blood during pregnancy, may be linked to cardiovascular disease in woman (Zechner, R et al. (1986) *Metabolism* 35:333-336). It has also been observed that diabetics, many of whom suffer in some degree from atherosclerotic diseases, display greatly elevated serum levels of Lp(a) (Bruckert, E., et al. (1990) *JAMA* 263:35-36).

Low levels of ascorbate have also been associated with a high incidence of cancer (Wright, L.C. et al. (1989) *Int. J. Cancer* 43:241-244) and atherosclerosis in diabetes mellitus patients (Som, S., et al. (1981) *Metabolism* 30:572-577). In all instances, serum concentrations of Lp(a) were elevated.

In addition to atherosclerosis and thrombosis in arteries, Lp(a) has also been linked to stenosis of vein grafts after coronary bypass surgery (Hoff, H., et al. (1988) *Circulation* 77:1238-1244). Similar problems of rapid occlusion of vessels have been observed in heart transplant patients.

For some time, general medical practice has focused on the role of LDL (the so called "bad cholesterol") in cardiovascular disease. Numerous studies have been published ostensibly linking cardiovascular disease with elevated levels of LDL. As a result, most therapies for the treatment and prevention of arteriosclerosis rely on drugs and methods for the reduction of serum levels of LDL's. Such therapies have had mixed results. The efficacy of such approaches to the problem of cardiovascular disease continues to be major source of debate.

There exists therefore a need for a drug therapy for reducing the binding of Lp(a) to vessel walls, for reducing the overall level of Lp(a) in the circulatory system and for promoting the release of existing deposits of Lp(a) on vessel walls.

The foregoing needs in the treatment and prevention of cardiovascular disease are met by the compositions of the present invention.

One object of the invention is the use of a composition comprising ascorbate and tranexamic acid for the manufacture of a medicament for therapeutic application in a method for treating occlusive cardiovascular disease to decrease the binding of lipoprotein(a) to blood vessel walls.

Another object of the invention is the use of a composition comprising ascorbate and tranexamic acid for the manufacture of a medicament for therapeutic application in a method for treating vessel explants prior to implantation to decrease the binding of lipoprotein(a) to interior walls of vessel explants.

Yet another object of the invention is the use of a composition comprising ascorbate and tranexamic acid for the manufacture of a medicament for therapeutic application in a method for treating transplanted vessels before, during or after transplant surgery to decrease the binding of lipoprotein(a) to interior walls of transplanted vessels.

A further object of the invention is the use of a composition comprising ascorbate and tranexamic acid for the manufacture of a medicament for therapeutic application to a hemodialysis solution in a method for treating occlusive cardiovascular disease resulting from renal failure to decrease the binding of lipoprotein-(a) to blood vessel walls.

A method for preservation of explanted tissues and organs is provided that reduces the risk of cardiovascular disease arising in such tissues and organs after implantation.

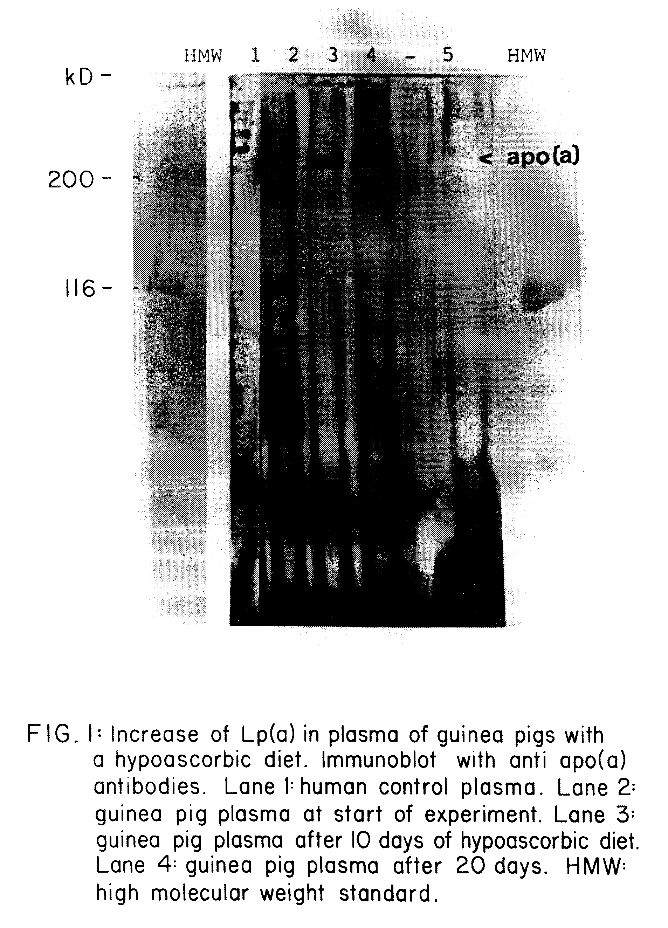

In order that the invention may be fully understood, reference is made on the accompanying drawings wherein Figure 1 is a photograph of an immunoblot of plasma from guinea pigs subjected to the treatments as described in Example 1.

Figure 2A is a photograph of an aorta of a guinea pig receiving an adequate amount of ascorbate from the test diet described in Example 1.

Figure 2B is a photograph of an aorta of a guinea pig after three weeks receiving a hypoascorbic diet as described in Example 1.

Figure 3 is a photograph of an immunoblot of plasma and tissue of guinea pigs subjected to the treatments as described in Example 2.

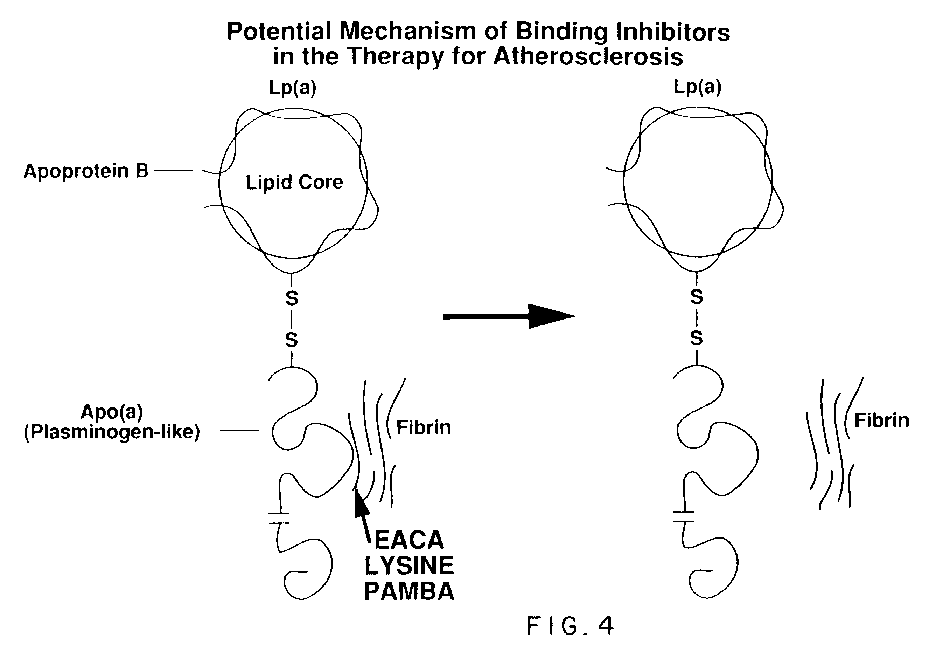

Figure 4 is a diagrammatic representation of the action of Lp(a) binding inhibitors to cause release of Lp(a) from fibrin fibers of an arterial wall.

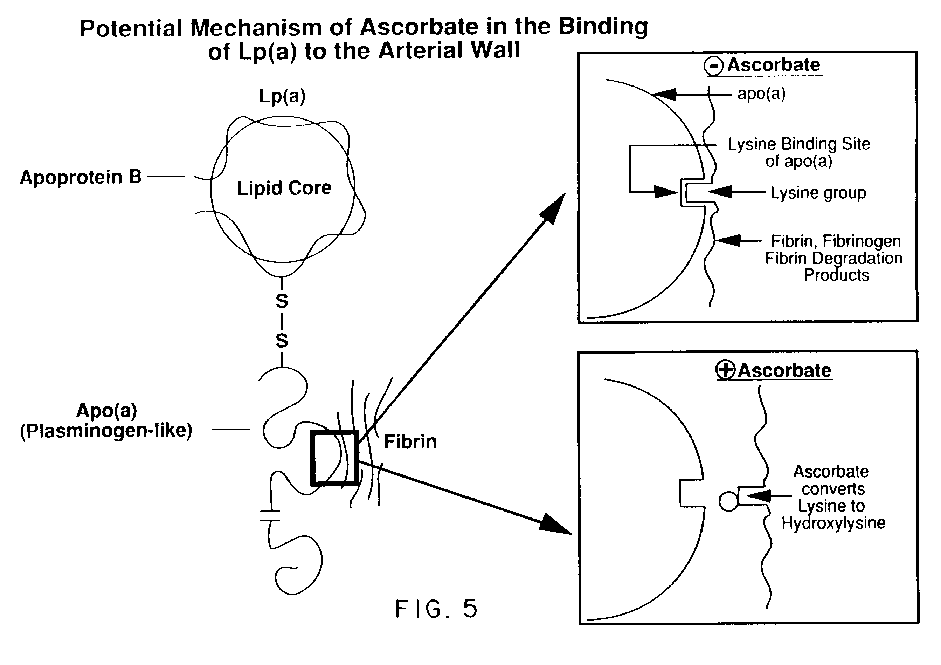

Figure 5 is a diagrammatic representation of the action of ascorbate to prevent association and reassociation of Lp(a) to an arterial wall.

The present invention is based in part on the discovery that animals which have lost the ability to produce ascorbate, such as higher primates and guinea pigs, uniformly produce Lp(a), whereas most animals which possess the ability to synthesize ascorbate generally do not produce Lp(a). Further, it has been found that ascorbate deficiency in humans and guinea pigs tends to raise Lp(a) levels and causes atherosclerosis by the deposition of Lp(a) in the arterial wall. Therefore, it appears that ascorbate administration lowers plasma Lp(a) levels.

It has also been discovered that tranexamic acid inhibits the binding of Lp(a) to components of the arterial wall, particularly to fibrinogen, fibrin and fibrin degradation products, herein identified as Lp(a) binding inhibitor. Thus, ascorbate and such a Lp(a) binding inhibitor is not only useful for the prevention of cardiovascular disease, but also for the treatment of such disease.

Some beneficial effects of ascorbate in the prevention and treatment of cardiovascular disease have been established. The present invention discloses the relation to and therapeutic use of ascorbate for reducing the effects of Lp(a), one of the most atherogenic lipoproteins, directly related to the development of atherosclerotic plaques. The beneficial effects of ascorbate suggest that ascorbate therapies would be useful in a variety of situations where occlusion of blood vessels by Lp(a) deposition is a problem. For instance, ascorbate not only appears useful in the treatment of endogenous atherosclerosis, but appears to be useful in transplantation of blood vessels and whole organs, where a combination of tissue damage to the transplant, such as oxidation, and high serum Lp(a) in the transplant recipient results in rapid occlusion of blood vessels in the transplant. Ascorbate may also be useful in the area of hemodialysis, where loss of ascorbate and other vitamins and trace elements from the blood of hemodialysis patients can result in increased serum levels of Lp(a) and thus increased risk of cardiovascular disease. Finally, it appears that ascorbate alone and in combination with Lp(a) binding inhibitors, specifically with plasmin competitors, may be therapeutically useful for treatment of the pathogenic effects of diabetes which is associated with elevated serum concentrations of Lp(a).

The present invention provides pharmaceutical compositions for both the treatment and prevention of cardiovascular disease in vivo; methods and compositions for the preservation of damage linked vessel occlusion in explanted tissues and organs, as well as methods and compositions for the prevention of hemodialysis-linked cardiovascular disease. Each of these embodiments is discussed in turn below.

General Applications

The present invention provides a pharmaceutical composition for the treatment and prevention of cardiovascular disease generally, particularly atherosclerosis, by administering to a subject a therapeutically effective amount of ascorbate and tranexamic acid which inhibits the binding of Lp(a) to blood vessel wall components, particularly to fibrin or fibrinogen. As used herein, the term "ascorbate" includes any pharmaceutically acceptable salt of ascorbate, including sodium ascorbate, as well as ascorbic acid itself. It is also considered advantageous to employ one or more antioxidants in the compositions of the present invention. The term antioxidant, as used throughout the specification and the claims, is intended to exclude ascorbate which has as one of its chemical properties a potent antioxidant effect. Representative antioxidants found useful in the practice of the invention include antioxidants such as tocopherol, carotene and related substances. Other substances used in the treatment of cardiovascular disease may be co-administered, including: vitamins; provitamins; trace elements; lipid-lowering drugs, such as hydroxy-methyl-glutaryl coenzyme A reductase inhibitors, nicotinic acid, fibrates, bile acid sequestrants; and mixtures of any two or more of these substances.

Where ascorbate and the Lp(a) binding inhibitor is utilized in the same composition, they may simply be mixed or may be chemically combined using synthesis methods well known in the art, such as compounds in which ascorbate and the inhibitor are covalently linked, or form ionically bound salts. In this form the ascorbate moiety may be particularly effective in preventing undesirable lipid peroxidation.

In the case of oral administration, a pharmaceutically acceptable and otherwise inert carrier may be employed. Thus, when administered orally, the active ingredients may be administered in tablet form. The tablet may contain a binder such as tragacanth, corn starch or gelatin; a disintegrating agent, such as alginic acid, and/or a lubricant such as magnesium stearate. If administration in liquid form is desired, use of sweetening and/or flavoring agents may be used. If administration is, by parenteral injection, in isotonic saline, a phosphate buffered solution or the like, may be used as a pharmaceutically acceptable carrier.

The advisability of using a Lp(a) binding inhibitor in treating cardiovascular disease will depend to some extent on the subject's general health, particularly with regard to hyperfibrinolytic conditions. Most Lp(a) binding inhibitors (except lysine) are used clinically to treat such conditions. As a result, monitoring of the subject's coagulation and fibrinolytic system is recommended before and during treatment for cardiovascular disease. Long-term administration of a Lp(a) binding inhibitor will require formulations in which the dosages of the Lp(a) binding inhibitor are in the lower ranges of the dosages disclosed in Table 1.

Prevention, as contrasted with treatment, of cardiovascular disease may be accomplished by oral or parenteral administration of ascorbate alone. Table 2 gives a range of ascorbate concentrations sufficient to lower the serum Lp(a) concentration. Preferably the prevention of the cardiovascular disease according to the invention is accomplished by use of a physical mixture of ascorbate and the Lp(a) binding inhibitor, or by use of a compound comprising covalently linked ascorbate with the binding inhibitor, which inhibit binding of Lp(a) to the arterial wall. A binding inhibitor or mixture of binding inhibitors may also be administered without ascorbate to prevent Lp(a)-associated cardiovascular disease.

To optimize the therapeutic effect of the release of Lp(a) from the blood vessel walls, the ascorbate and the binding inhibitors described above may be separately administered. Further optimization of therapeutic effect can be gained by using a time release composition to achieve relatively constant serum concentrations of the composition through time.

Coronary Bypass Applications

As discussed above, recurrence of cardiovascular disease after bypass surgery is a frequent problem. Physicians often observe that the veins used to replace occluded arteries become rapidly occluded themselves after implantation, often requiring the patient to undergo successive surgical episodes to replace clogged bypasses. While the utility of the present invention is not dependent on the veracity of any theory, it appears that the rapid occlusion observed in many individual's results from a combination of the patient's pre-existing elevated levels of Lp(a) and injury to the bypass veins during transplantation, particularly as a result oxidative damage during explantation. This damage makes binding of Lp(a) to the vessel interior easier. Further, Lp(a) has been detected in abundance in reoccluded by-pass veins after coronary bypass surgery. See, Cushing, et al. (1989) Atherosclerosis 9:593-603. Lp(a) is now known to be the most significant factor for reocclusion of bypass veins. See, Hoff, H, et al. (1988) Circulation 77:1238-1244. Thus, a further embodiment of this invention includes using the pharmaceutical composition of the present invention to lower the bypass patient's Lp(a) before, during and after surgery while at the same using a solution containing the composition to rinse and store the bypass veins until such time as the veins are implanted into the recipient, thereby reducing oxidative damage that can make Lp(a) binding more likely after implantation.

The treatment protocols for the bypass patient generally follow those described above for the treatment of pre-existing cardiovascular disease. The composition of the pharmaceutical composition will generally include ascorbate, one or more binding inhibitors, one or more antioxidants and one or more lipid lowering drugs as enumerated and in the dosages disclosed in Table 1. Of course, the level of dosage will depend on disease severity. Further, the constituents of the composition can be combined just as described above, can be administered either orally or parenterally and can be combined with a pharmaceutically acceptable carrier.

Table 1

| DOSAGE RANGES OF COMPONENTS IN COMPOSITIONS OF THE PRESENT INVENTION | | |
|---|---|---|
| | Oral Administration | Parenteral Administration |
| Ascorbate: | 5 - 2500 mg/kg BW/d | 25 - 2500 mg/kg BW/d |
| Binding inhibitors: | | |
| Tranexamic acid | 1 - 100 mg/kg BW/d | same |
| Antioxidants: | | |
| Tocopherol | 0.1 - 20 IU/kg BW/d | same |
| Carotene | 100 - 1000 IU/kg BW/d | same |
| Lipid Lowering Drugs: | | |
| Nicotinic Acid | 1 - 300 mg/kg BW/d | |
| HMG-CoA | 0.1 - 10 mg/kg BW/d | |
| Fibrates | 0.1 - 20 mg/kg BW/d | |
| Probucol | 0.1 - 20 mg/kg BW/d | |
| Bile Acid Sequestrants | 10 - 400 mg/kg BW/d | |

Turning now to vessel treatment and storage, it is important to provide an in vitro environment which minimizes vessel injury. It appears that vessel injury can be reduced by the addition of a combination of ascorbate, binding inhibitor and antioxidants to the solution in which the vessels are normally stored. A range of effective concentrations of these constituents in solution is disclosed in Table 2. The general aspects of live vessel preservation and storage prior to implantation are well known in the art.

Table 2

| CONCENTRATIONS OF COMPONENTS IN SOLUTIONS OF THE PRESENT INVENTION | |
|---|---|
| Ascorbate | 50 - 5000 mg/l |
| Binding inhibitor | |
| Tranexamic Acid | 1 - 300 mg/l |
| Antioxidants | |
| Tocopherol | 1 - 1000 mg/l |
| Carotene | 0.1 - 100 mg/l |

Applications in Organ Transplants

It has also been found that the composition of the present invention is effective in preventing cardiovascular vascular disease from occurring in transplanted organs that have been otherwise successfully implanted in an organ recipient, particularly in the case of the heart.

As with occlusion of transplanted veins after bypass surgery, a transplanted heart free of any substantial arterial occlusion may suffer accelerated atherosclerosis after implantation. It appears that the mechanism described for occlusion of transplanted vessels applies equally to the heart itself as a whole, namely that the heart muscle itself, as well as the interiors of the arterial walls become damaged, making the arteries of the heart more prone to binding with Lp(a). Because the organ recipient often presents elevated serum concentration of Lp(a), particularly after surgery (see, Maeda, S. et al. (1989) *Atherosclerosis* 78:145-150), atherosclerosis can proceed at an accelerated rate.

Treatment follows along the same line as that described for bypass surgery. Damage to the organ itself is minimized by placing the organ in a solution containing a mixture of ascorbate, binding inhibitors, and antioxidants in an otherwise standard storage solution. Concentration ranges for the various components of the present composition in the final solution are disclosed in Table 2. Because of the oxidative cellular damage during extended periods of explantation, the concentration of antioxidants should be in the higher range of dosages disclosed in Table 1. The standard storage solution itself is well known in the art. Storage of the organ in a solution containing the present composition will tend to minimize damage to arterial walls, thereby providing fewer places for Lp(a) to bind.

Of course, patient treatment is also desireable. If the organ recipient suffers from some degree of atherosclerosis at the time of organ transplant, the protocol and drug described above generally for the treatment of atherosclerosis should be employed. If, however, the patient does not suffer from atherosclerosis, use of the drug and protocol described above for prevention of atherosclerosis is desired. In all cases, the lowest dosages of ascorbate should be employed in the drug composition since ascorbate has an immune stimulatory effect.

Applications in Hemodialysis Treatment

It is well known that patients who suffered renal failure and require regular dialysis treatment to cleanse the blood of metabolic waste products are also at an increased risk for cardiovascular disease. It appears that the reason for this may be a depletion of ascorbate, vitamins in general and other essential substances from the blood supply during the hemodialytic process. As described more fully above, the loss of ascorbate would result in greater injury to the interior of the artery walls over time and may also result in the production of elevated Lp(a) levels in the blood serum.

As can be readily appreciated, the composition of the present invention can be applied both to the patient and the hemodialysis solution to prevent and control hemodialysis-related cardiovascular disease. Turning first to the hemodialysis solution, it is presently considered desirable to add a composition comprising a combination of ascorbate, binding inhibitors and antioxidants to the solution to produce concentrations of these compounds in solution in the range of concentrations provided in Table 2.

It has been determined that advantageous results can be achieved by carrying out treatment of the dialysis patient in addition to modification of the hemodialysis solution. Treatment should follow the drug and protocols set forth in detail above for the treatment of a preexisting atherosclerotic condition.

Applications in Treatment of Diabetes

The composition of the present invention is also useful in the treatment of the pathological effects of diabetes mellitus. In diabetes mellitus, pathological charges in the arteries frequently lead to clinical symptoms or complete failure in various organs such as the kidney, eye and peripheral circulation system. Therefore, one therapeutic focus in diabetes mellitus is the treatment of diabetic angiopathy.

It appears that glucose competitively inhibits the physiologic uptake of ascorbate in different cell systems of the body, including the arterial wall. Kapeghian, et al. (1984) *Life Sci.* 34:577. Such damage to arterial walls creates binding sites for Lp(a). Further, Lp(a) has been found to be elevated in the blood serum of diabetic patients. The atherogenic process is perhaps therefore accelerated by the combination of damaged arteries and elevated Lp(a). Therefore, the present invention discloses that ascorbate alone or in combination with at least one binding inhibitor has therapeutic value in treating diabetes-related atherosclerosis.

Thus, another embodiment of the present invention is the use of a composition for treating the pathogenic effects of diabetes mellitus, particularly with regard to atherosclerotic conditions.

The treatment protocol involves the oral or parenteral administration of a pharmaceutical composition comprised of ascorbate, one or more binding inhibitors and one or more antioxidants. Dosages for a course of treatment are provided in Table 1. The dosage of ascorbate should preferably fall within the higher range, thereby increasing its chance of cellular uptake in the presence of high serum levels of glucose.

Experimental

Having disclosed the preferred embodiment of the present invention, the following examples are provided by way of illustration.

Example 1

Because of its metabolic similarity to man, with respect to the metabolism of ascorbate and Lp(a), the guinea pig was used in this example.

No study has been previously reported in the guinea pig to identify the lipoprotein involved as risk factors in plasma and as constituents of the atherosclerotic plaque.

Three female Hartly guinea pigs with an average weight of 800 g and an approximate age of 1 year were studied. One animal received an extreme hypoascorbic diet with 1 mg ascorbate/kg body weight/day (kg BW/d). Another animal received 4 mg/kg BW/d. The third animal served as a control receiving 40 mg ascorbate/d.

Blood was drawn by ear puncture from the anesthetized animals and collected into EDTA containing tubes at the beginning, after 10 days, and after 3 weeks, when the animals were sacrificed. Plasma was stored at -80°C until analyzed. Lp(a) was detected in the plasma of the guinea pigs by use of SDS-polyacrylamide gels according to Neville ((1971) *J. Biol. Chem.* 246:6328-6334) followed by Western blotting according to Beisiegel, et al. ((1982) *J. Biol. Chem.* 257:13150-13156). Forty $\mu$l of plasma and 20 mg of arterial wall homogenate were applied in delipidated form per lane of the gel. The immunodetection of apo(a) was performed using a polyclonal anti-human apo(a) antibody (Immuno, Vienna, Austria) followed by a rabbit anti-sheep antibody (Sigma) and the gold-conjugated goat anti-rabbit antibody with subsequent silver enhancement (Bio-Rad). The determinations of cholesterol and triglycerides were done at California Veterinary Diagnostics (Sacramento) using the enzyme assay of Boehringer Mannheim. The amount of plasma ascorbate was determined by the dinitrophenylhydrazine method according to Schaffer, et al. (-(1955) *J. Biol. Chem.* 212:59).

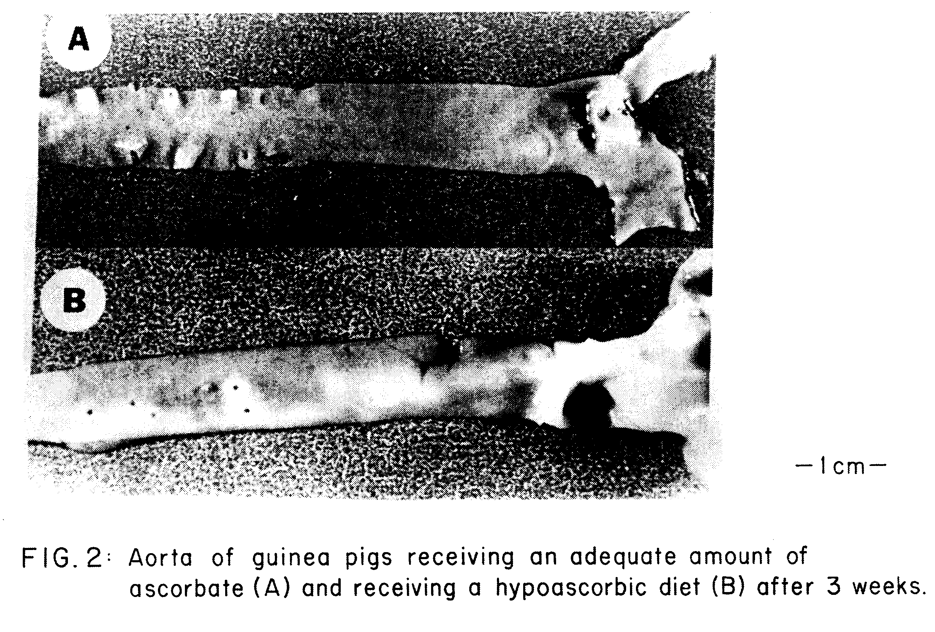

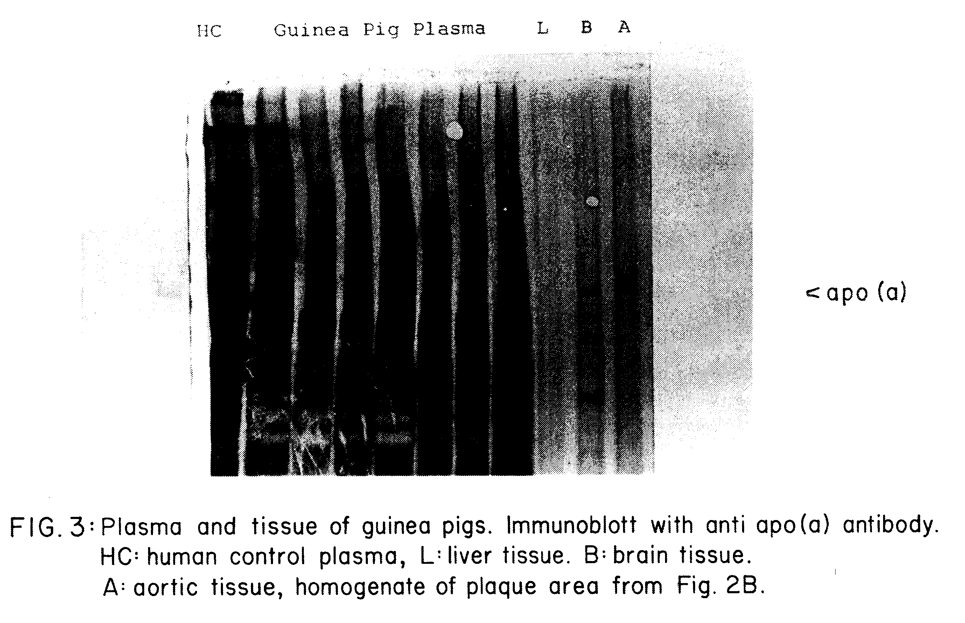

Vitamin C deficiency in the diet led to an increase of Lp(a) in the plasma of the guinea pig, as indicated by a clear band in the immunoblot of the plasma after 10 and 20 days of a hypoascorbic diet (Figure 1). At necropsy the animals were anesthetized with metophase and exsanguinated. The aorta, heart and various other organs were taken for biochemical and histological analysis. The aorta was excised, the adventitial fat carefully removed, and the vessel was opened longitudinally. Subsequently the aorta was placed on a dark metric paper and a color slide was taken. The picture was projected and thereby magnified by an approximate factor of 10. The circumference of the ascending aorta, the aortic arch and thoracic aorta, as well as the atherosclerotic lesions in this area, were marked and measured with a digitalized planimetry system. The degree of atherosclerosis was expressed by the ratio of plaque area in relation to the total aortic area defined. The difference in the three one-year old animals of the experiment was significant and pronounced lesions were observed in the ascending aorta and the arch of the vitamin C deficient animal (Figure 2B).

Example 2

To confirm the data obtained in Example 1, a second guinea pig experiment was conducted, using 33 male animals with a mean weight of 550 g and an approximate age of 5 months. One group of 8 animals served as a control and received 40 mg ascorbate/kg BW/d (group A). To induce hypoascorbemia 16 animals were fed 2 mg ascorbate/kg BW/d (group B). Group A and half of the animals of group B (progression sub-group) were sacrificed after 5 weeks as described above. Half of group B was kept for 2 more weeks, receiving daily intraperitoneal injection of 1.3 g Na-ascorbate/kg BW/d as a daily intra peritoneal injection with the intention to reduce the extent of atherosclerosis lesions. After this regression period these animals also were sacrificed.

Plasma ascorbate levels were negatively correlated with the degree of the atherosclerotic lesion. Total cholesterol levels increased significantly during vitamin C deficiency (Table 3).

The aortas of the guinea pigs receiving a sufficient amount of ascorbate were essentially plaque free, with minimal thickening of the intima in the ascending region. In contrast, the ascorbate-deficient animals exhibited fatty streak-like lesions, covering most parts of the ascending aorta and the aortic arch. In most cases the branching regions of the intercostal arteries of the aorta exhibited similar lipid deposits. The difference in the percentage of lesion area between the control animals and the hypoascorbic diet animals was 25% deposition of lipids and lipoproteins in the arterial wall.

Table 3

| MEAN PLASMA PARAMETERS OF THE DIFFERENT GROUPS IN RELATION TO THE AREA OF AORTIC LESIONS | | | |
|---|---|---|---|
| | Control | Scurvy (progress) | Regression (after Scurvy) |
| Plasma Cholesterol (mg/dl) | 39 | 54 | 33 |
| Total Plasma Ascorbic Acid ($\mu$g/ml) | 5.03 | 3.01 | 20.64 |
| Atheroscl. Lesion (Percent of Aorta Thorac. Surface) | -- | 25 | 19 |

The effectiveness of an inhibitor may be identified first by adding molar amounts of the possible inhibitor at a little larger, by approximately 5 times, the amount of $\epsilon$-aminocaproic acid found in the earlier study. If, at this concentration, a possible inhibitor is found to inhibit the agglutination, studies are made at lower concentrations, to determine the concentration that has a 50% inhibitory effect.

Example 3

Human arterial wall was obtained post mortem from the aorta ascendens. The tissue showed homogenous intimal thickening (early atherosclerotic lesion). It was cut into pieces, with 100 mg samples of the cut up tissue each homogenized in a glass potter for 1 minute in 2.5 ml of the following solutions in PBS (Dulbeco):

| NaAscorbate | 50 mg/ml |
|---|---|
| EACS | 50 mg/ml |
| Tranexamic Acid | 50 mg/ml |
| NaAscorbate + Tranexamic Acid | 50 mg/ml |

Results of this treatment are disclosed in Table 4 and show that, compared to the control solution, a considerable amount of Lp(a) was released from the interior arterial wall.

## Table 4

### Lp(a) RELEASED FROM HUMAN AORTA
### IN RELATION TO SPECIFIC BINDING INHIBITORS

By now it is apparent that the compositions of the present invention meet longstanding needs in the field of prevention and treatment of cardiovascular disease.

### Claims

1. Use of a composition comprising ascorbate and tranexamic acid for the manufacture of a medicament for therapeutic application in a method for treating occlusive cardiovascular disease to decrease the binding of lipoprotein(a) to blood vessel walls.

2. Use of a composition comprising ascorbate and tranexamic acid for the manufacture of a medicament for therapeutic application in a method for treating vessel explants prior to implantation to decrease the binding of lipoprotein(a) to interior walls of vessel explants.

3. Use of a composition comprising ascorbate and tranexamic acid for the manufacture of a medicament for therapeutic application in a method for treating transplanted vessels before, during or after transplant surgery to decrease the binding of lipoprotein(a) to interior walls of transplanted vessels.

4. Use of a composition comprising ascorbate and tranexamic acid for the manufacture of a medicament for therapeutic application to a hemodialysis solution in a method for treating occlusive cardiovascular disease resulting from renal failure to decrease the binding of lipoprotein(a) to blood vessel walls.

### Patentansprüche

1. Verwendung einer Zusammensetzung, die Ascorbat und Tranexamsäure umfaßt, für die Herstellung eines Medikaments zur therapeutischen Anwendung bei einem Verfahren zum Behandeln einer okklusiven Herz-Kreislauf-Erkrankung, um die Bindung von Lipoprotein(a) an Blutgefäßwänden zu verringern.

**2.** Verwendung einer Zusammensetzung, die Ascorbat und Tranexamsäure umfaßt, für die Herstellung eines Medikaments zur therapeutischen Anwendung bei einem Verfahren zum Behandeln von Gefäßexplantaten vor der Implantierung, um die Bindung von Lipoprotein(a) an inneren Wänden von Gefäßexplantaten zu verringern.

**3.** Verwendung einer Zusammensetzung, die Ascorbat und Tranexamsäure umfaßt, für die Herstellung eines Medikaments zur therapeutischen Anwendung bei einem Verfahren zum Behandeln von transplantierten Gefäßen vor, während oder nach der Transplantationschirurgie zum Verringern der Bindung von Lipoprotein(a) an inneren Wänden von transplantierten Gefäßen.

**4.** Verwendung einer Zusammensetzung, die Ascorbat und Tranexamsäure umfaßt, für die Herstellung eines Medikaments zur therapeutischen Anwendung bei einer Hämodialyselösung bei einem Verfahren zum Behandeln einer okklusiven Herz-Kreislauf-Erkrankung, die aus Nierenversagen resultiert, um die Bindung von Lipoprotein(a) an Blutgefäßwänden zu verringern.

**Revendications**

**1.** Utilisation d'une composition comprenant de l'ascorbate et de l'acide tranexamique pour la fabrication d'un médicament, pour une application thérapeutique dans une méthode pour le traitement d'une maladie cardiovasculaire occlusive, pour diminuer la liaison de la lipoprotéine(a) aux parois des vaisseaux sanguins.

**2.** Utilisation d'une composition comprenant de l'ascorbate et de l'acide tranexamique pour la fabrication d'un médicament, pour une application thérapeutique dans une méthode pour le traitement d'explants de vaisseaux avant l'implantation, pour diminuer la liaison de la lipoprotéine(a) aux parois internes des explants de vaisseaux.

**3.** Utilisation d'une composition comprenant de l'ascorbate et de l'acide tranexamique pour la fabrication d'un médicament, pour une application thérapeutique dans une méthode pour le traitement de vaisseaux transplantés avant, pendant ou après une chirurgie de transplantation, pour diminuer la liaison de la lipoprotéine(a) aux parois internes des vaisseaux transplantés.

**4.** Utilisation d'une composition comprenant de l'ascorbate et de l'acide tranexamique pour la fabrication d'un médicament, pour une application thérapeutique à une solution d'hémodialyse dans une méthode pour le traitement d'une maladie cardiovasculaire occlusive résultant d'une défaillance rénale, pour diminuer la liaison de la lipoprotéine(a) aux parois des vaisseaux sanguins.

FIG. I: Increase of Lp(a) in plasma of guinea pigs with a hypoascorbic diet. Immunoblot with anti apo(a) antibodies. Lane 1: human control plasma. Lane 2: guinea pig plasma at start of experiment. Lane 3: guinea pig plasma after 10 days of hypoascorbic diet. Lane 4: guinea pig plasma after 20 days. HMW: high molecular weight standard.

FIG. 2: Aorta of guinea pigs receiving an adequate amount of ascorbate (A) and receiving a hypoascorbic diet (B) after 3 weeks.

EP 0 532 706 B1

HC    Guinea Pig Plasma    L  B  A

< apo (a)

FIG. 3: Plasma and tissue of guinea pigs. Immunoblott with anti apo(a) antibody.
HC: human control plasma, L: liver tissue. B: brain tissue.
A: aortic tissue, homogenate of plaque area from Fig. 2B.

EP 0 532 706 B1

# Potential Mechanism of Binding Inhibitors in the Therapy for Atherosclerosis

FIG. 4

EP 0 532 706 B1

# Potential Mechanism of Ascorbate in the Binding
## of Lp(a) to the Arterial Wall

Lp(a)

Apoprotein B ——

Lipid Core

S
|
S

Apo(a)
(Plasminogen-like) ——

Fibrin

FIG. 5

⊖ Ascorbate

apo(a)

Lysine Binding Site
of apo(a)

Lysine group

Fibrin, Fibrinogen
Fibrin Degradation
Products

⊕ Ascorbate

Ascorbate
converts
Lysine to
Hydroxylysine

EP 0 532 706 B1